# EUROPÄISCHE PATENTANMELDUNG

(11) **EP 0 264 050 A2**
(43) Veröffentlichungstag der Anmeldung: **20.04.1988**
(21) Anmeldenummer: 87114553.8
(22) Anmeldetag: 06.10.1987
(51) Int. Cl.: C07D 215/56, C07D 401/04, C07D 409/14, A61K 31/47

(54) **7-Amino-1-cyclopropyl-8-chlor-6-fluor-1, 4-dihydro-4-oxo-3-chinolincarbonsäuren, Verfahren zu ihrer Herstellung sowie diese enthaltende antibakterielle Mittel**

(30) Priorität: 16.10.1986 DE 3635218
(71) Anmelder: BAYER AG, 51368 Leverkusen (DE)
(72) Erfinder: Petersen, Uwe, Dr., D-5090 Leverkusen 1 (DE); Grohe, Klaus, Dr., D-5068 Odenthal (DE); Schriewer, Michael, Dr., D-5068 Odenthal (DE); Haller, Ingo, Dr., D-5600 Wuppertal 1 (DE); Zeiler, Hans-Joachim, Dr., D-5600 Wuppertal 1 (DE); Metzger, Karl-Georg, Dr., D-5600 Wuppertal 1 (DE); Endermann, Rainer, Dr., D-5600 Wuppertal 1 (DE)

(57) **Zusammenfassung**

Die Erfindung betrifft neue 7-Amino-1-cyclopropyl-8-chlor-6-fluor-1,4-dihydro-4-oxo-3-chinolincarbonsäuren der Formel (I) 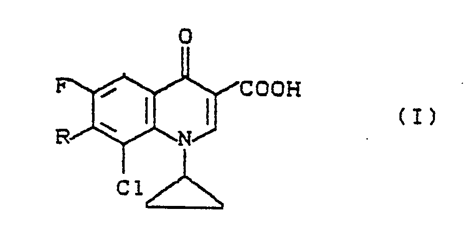 in welcher
R, R¹, R² und R³ die in der Beschreibung angegebene Bedeutung haben, Verfahren zu ihrer Herstellung sowie diese enthaltende antibakterielle Mittel.

## Beschreibung

Die vorliegende Erfindung betrifft neue 7-Amino-1-cyclopropyl-8-chlor-6-fluor-1,4-dihydro-4-oxo-3-chinolincarbonsäuren, Verfahren zu ihrer Herstellung sowie diese enthaltende antibakterielle Mittel.

Gefunden wurden die neuen 7-Amino-1-cyclopropyl-8-chlor-6-fluor-1,4-dihydro-4-oxo-3-chinolincarbonsäuren der Formel (I), 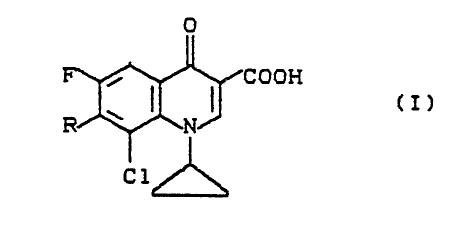 in welcher
R für 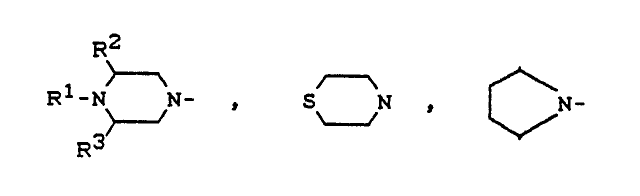 oder 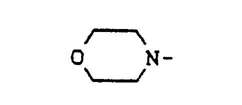
steht, wobei
R¹ H, CH₃, C₂H₅ oder HO-CH₂-CH₂- bedeutet und
R² für H, CH₃, C₂H₅, 2-Thienyl oder Phenyl und
R³ für H oder CH₃ stehen,
mit der Maßgabe, daß R nicht für 1-Piperazinyl, 4-Methyl-1-piperazinyl oder 3-Methyl-1-piperazinyl stehen kann,
und deren pharmazeutisch verwendbaren Hydrate, Säureadditionssalze, Alkali-, Erdalkali- und Guanidiniumsalze, die eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen R für 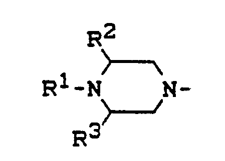 mit den oben für R¹, R² und R³ angegebenen Bedeutungen steht und für die die oben angegebene Maßgabe gilt.

Besonders bevorzugt sind auch die Säureadditionssalze der erfindungsgemäßen Verbindungen.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Wirkstoffe können sowohl als Racemate als auch als enantiomerenreine Verbindungen vorliegen.

Weiterhin wurde gefunden, daß man die Verbindungen der Formel (I) erhält, wenn man die 8-Chlor-1-cyclopropyl-6,7-difluor-1,4-dihydro-4-oxo-3-chinolincarbonsäure der Formel (II), 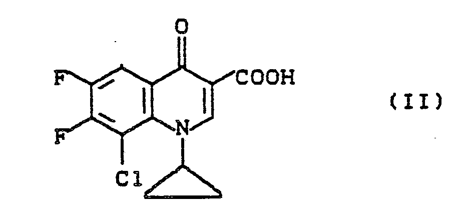 mit Aminen der Formel (III),
R - H (III)
in welcher
R die oben angegebene Bedeutung hat,
gegebenenfalls in Gegenwart von Säurebindern umsetzt (Methode A).

Erfindungsgemäße Verbindungen der Formel (I) können auch erhalten werden, indem man eine 7-(1-Piperazinyl)-3-chinoloncarbonsäure der Formel (IV), 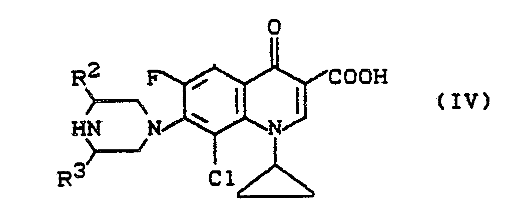 in welcher
R² und R³ die oben angegebene Bedeutung haben,
mit Verbindungen der Formel (V)
R¹X (V)
in welcher
R¹ die oben angegebene Bedeutung hat, jedoch nicht Wasserstoff sein kann, und
X Fluor, Chlor, Brom, Iod, bedeutet,
gegebenenfalls in Gegenwart von Säurebindern umsetzt (Methode B).

Verwendet an bei der Umsetzung nach Methode A 2-Ethylpiperazin und 8-Chlor-1-cyclopropyl-6,7-difluor-1,4-dihydro-4-oxo-3-chinolincarbonsäure als Ausgangsstoffe, so kann der Reaktionsablauf durch das folgende Formelschema wiedergegeben werden:

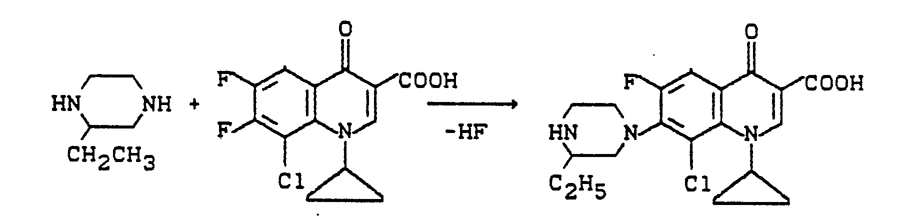

Verwendet man bei der Umsetzung nach Methode B Ethyliodid und 8-Chlor-1-cyclopropyl-6-fluor-1,4-dihydro-4-oxo-7-(1-piperazinyl)-3-chinolincarbonsäure als Ausgangsstoffe, so kann der Reaktionsablauf durch das folgende Formelschema wiedergegeben werden:

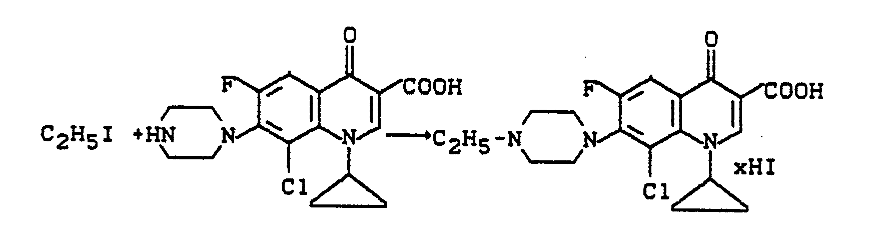

Die als Ausgangstoff nach Methode A verwendbare 8-Chlor-1-cyclopropyl-6,7-difluor-1,4-dihydro-4-oxo-3-chinolincarbonsäure der Formel (II) ist aus der Deutschen Offenlegungsschrift 3 420 743 bereits bekannt.

Die als Ausgangsstoffe verwendeten Amine (III) sind bekannt oder können nach literaturbekannten Verfahren erhalten werden [US 4 166 180, J. Med. Chem. 26, 1116 (1983)]. Als Beispiele seien genannt:
Morpholin, Thiomorpholin, Pyrrolidin, N-Ethylpiperazin, N-(2-Hydroxethyl)-piperazin 1,2-Dimethylpiperazin, cis- und trans-2,6-Dimethylpiperazin, 2-Ethylpiperazin, 2-(2-Thienyl)-piperazin, 2-Phenylpiperazin.

Die als Ausgangstoffe verwendeten Verbindungen der Formel (IV) sind teilweise aus der Deutschen Offenlegungsschrift 3 420 743 bekannt oder sind Gegenstand dieser Erfindung. Als Beispiele seien genannt: 8-Chlor-1-cyclopropyl-6-fluor-1,4-dihydro-4-oxo-7-(1-piperazinyl)-3-chinolincarbonsäure, 8-Chlor-1-cyclopropyl-6-fluor-1,4-dihydro-7-(3-methyl-1-piperazinyl)-4-oxo-3-chinolincarbonsäure.

Die als Ausgangsstoffe verwendeten Verbindungen der Formel (V) sind bekannt. Als Beispiel seien genannt:
Methyliodid, Methylbromid, Ethyliodid, Ethylbromid, Ethylchlorid, 2-Hydroxyethylchlorid.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykol monomethylether, Acetonitril oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine and Amidine. Als besonders geeignet seien im einzelnen genannt: Triethylamin, 1,4-Diazabicyclo[2,2,2]-octan (DABCO), 1,8-Diazabicyclo[5,4,0]-undec-7-en (DBU) oder überschüssiges Amin (III).

Die Reaktionstemperaturen können in einem größeren Bereich variiert werden. Im allgemeinen arbeitet man zwischen etwa 20 und 200° C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise 1 und 10 bar.

Bei der Durchführung des erfindungsgemäßen Verfahrens nach Methode A setzt man auf 1 Mol der Carbonsäure (II) 1 bis 15 Mol, vorzugsweise 1 bis 6 Mol des Amins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazbicyclo-[5,4,0]undec-7-en (DBU).

Die Reaktionstemperaturen können in einem größeren Bereich variiert werden. Im allgemeinen arbeitet man zwischen etwa 20 und etwa 180 °C, vorzugsweise zwischen 40 und 110°C.

Die Umsetzung kann bei Normaldruck, aber auch bei erhöhtem Druck durchgeführt werden. Im allgemeinen arbeitet man bei Drucken zwichen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Außer den in den Beispielen aufgeführten Verbindungen seien als neue Wirkstoffe im einzelnen genannt:
8-Chlor-1-cyclopropyl-6-fluor-1,4-dihydro-7-(3,4-dimethyl-1-piperazinyl)-4-oxo-3-chinolincarbonsäure,
8-Chlor-1-cyclopropyl-7-(4-ethyl-3-methyl-1-piperazinyl)-6-fluor-1,4-dihydro-4-oxo-3-chinolincarbonsäure,
8-Chlor-1-cyclopropyl-6-fluor-1,4-dihydro-7-[4-(2-hydroxyethyl)-3-methyl-1-piperazinyl]-4-oxo-3-chinolincarbonsäure,
8-Chlor-1-cyclopropyl-6-fluor-1,4-dihydro-4-oxo-7-(3,4,5-trimethyl-1-piperazinyl)-3-chinolcarbonsäure,
8-Chlor-1-cyclopropyl-7[(4-ethyl-3,5-dimethyl-1-piperazinyl)-6-fluor-1,4-dihydro-4-oxo-3-chinolincarbonsäure,
8-Chlor-1-cyclopropyl-6-fluor-1,4-dihydro-7-(4-methyl-3-thienyl-1-piperazinyl)-4-oxo-3-chinolincarbonsäure,
8-Chlor-1-cyclopropyl-6-fluor-1,4-dihydro-7-(trans-3,5-dimethyl-1-piperazinyl)-4-oxo-3-chinolincarbonsäure,
8-Chlor-1-cyclopropyl-7-(3-ethyl-1-piperazinyl)-6-fluor-1,4-dihydro-4-oxo-3-chinolincarbonsäure,
8-Chlor-1-cyclopropyl-7-(3-ethyl-4-methyl-1-piperazinyl)-6-fluor-1,4-dihydro-4-oxo-3-chinolincarbonsäure.

Die folgenden Beispiele erläutern die Erfindung:

### Beispiel 1

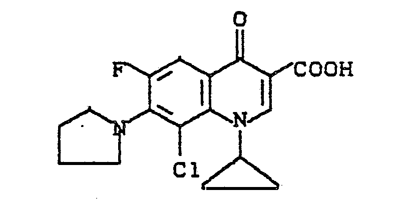

3 g (10 mmol) 8-Chlor-1-cyclopropyl-6,7-difluor-1,4-dihydro-4-oxo-3-chinolincarbonsäure werden in einer Mischung aus 20 ml Dimethylformamid in Gegenwart von 1,1 g (10 mmol) 1,4-Diazabicyclo[2,2,2]octan mit 0,8 g (11 mmol) Pyrrolidin 1 Stunde unter Rückfluß erhitzt. Die Mischung wird eingedampft, der Rückstand, mit Wasser versetzt, auf pH 4-5 eingestellt, das ausgefallene Produkt abgesaugt, getrocknet und aus Glykolmonomethylether umkristallisiert. Man erhält 2,8 g (80% der Theorie) 8-Chlor-1-cyclopropyl-6-fluor-1,4-dihydro-4-oxo-7-(1-pyrrolidinyl)-3-chinolincarbonsäure, die sich ab etwa 235°C zersetzt und bei 285-287°C geschmolzen ist.

### Beispiel 2

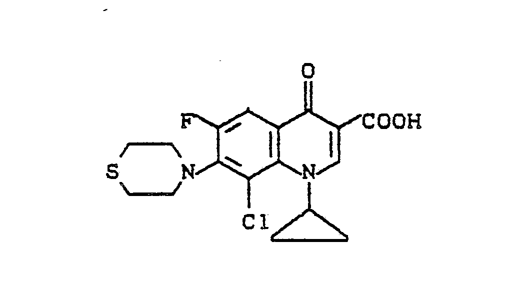

Führt man die Umsetzung analog Beispiel 1 mit Thiomorpholin durch, dann erhält man 8-Chlor-1-cyclopropyl-6-fluor-1,4-dihydro-4-oxo-7-(tetrahydro-1,4-thiazin-4-yl)-3-chinolincarbonsäure vom Schmelzpunkt 200-207° (unter Zersetung).

### Beispiel 3

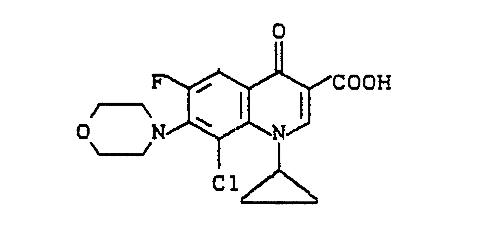

Führt man die Umsetzung analog Beispiel 1 mit Morpholin durch, dann erhält man 8-Chlor-1-cyclopropyl-6-fluor-1,4-dihydro-7-morpholino-4-oxo-3-chinolincarbonsäure vom Schmelzpunkt 204-205°C (unter Zersetzung).

### Beispiel 4

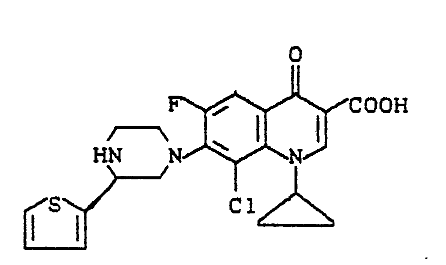

Führt man die Umsetzung analog Beispiel 1 mit 2-(2-Thienyl-)piperazin durch, dann erhält man 8-Chlor-1-cyclopropyl-6-fluor-1,4-dihydro-4-oxo-7-[3-(2-thienyl)-1-piperazinyl]-3-chinolincarbonsäure vom Schmelzpunkt 190-195°C (unter Zersetzung).

### Beispiel 5

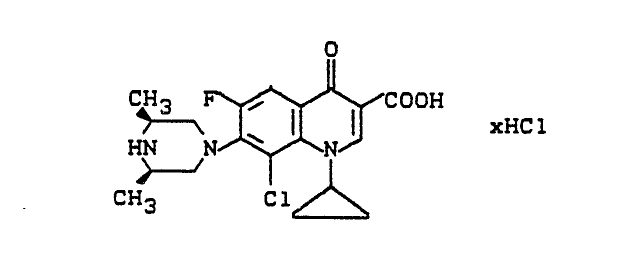

6 g (20 mmol) 8-Chlor-1-cyclopropyl-6,7-difluor-1,4-dihydro-4-oxo-3-chinolincarbonsäure werden in einer Mischung aus 40 ml Acetonitril und 20 ml Dimethylformamid in Gegenwart von 2,2 g (20 mmol) 1,4-Diazabicyclo[2.2.2]octan mit 3 g (26 mmol) cis-2,6-Dimethylpiperazin versetzt und 4 Stunden unter Rückfluß erhitzt. Die Mischung wird eingeengt, der Rückstand mit Wasser verrührt und mit 2n-Salzsäure auf pH 6-7 eingestellt. Die ausgefallene 8-Chlor-1-cyclopropyl-6-fluor-1,4-dihydro-7-(cis-3,5-dimethyl-1-piperazinyl)-4-oxo-3-chinolincarbonsäure (Betain) (5,4 g, Schmelzpunkt 207-211°C unter Zersetzung) wird abgesaugt, mit Wasser gewaschen und in 35 ml 6n-Salzsäure heiß gelöst. Die Lösung wird filtriert und das Filtrat mit 400 ml Ethanol versetzt. Beim Abkühlen fällt das Produkt aus, das mit Ethanol gewaschen und bei 80°C im Hochvakuum getrocknet wird. Man erhält 2,4 g (28 % der Theorie) 8-Chlor-1-cyclopropyl-6-fluor-1,4-dihydro-7-(cis-3,5-dimethyl-1-piperazinyl)-4-oxo-3-chinolincarbonsäure-Hydrochlorid vom Schmelzpunkt 317°C (unter Zersetzung).

### Beispiel 6

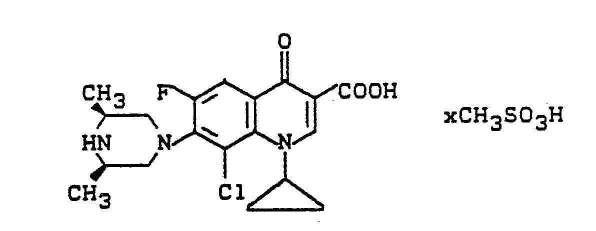

3 g (7,6 mmol) 8-Chlor-1-cyclopropyl-6-fluor-1,4-dihydro-7-(cis-3,5-dimethyl-1-piperazinyl)-4-oxo-3-chinolincarbonsäure (Betain) werden in 70 ml Ethanol bei etwa 70°C suspendiert und mit 1 g Methansulfonsäure versetzt. Unter Rühren wird abgekühlt, das erhaltene Salz abgesaugt, mit Ethanol gewaschen und bei 80°C im Vakuum getrocknet. Man erhält 2,6 g (70 % der Theorie) 8-Chlor-1-cyclopropyl-6-fluor-1,4-dihydro-7-(cis-3,5-dimethyl-1-piperazinyl)-4-oxo-3-chinolincarbonsäure-Methansulfonat vom Schmelzpunkt 265-274°C (Zersetzung). Das Salz löst sich sehr leicht in Wasser.

### Beispiel 7

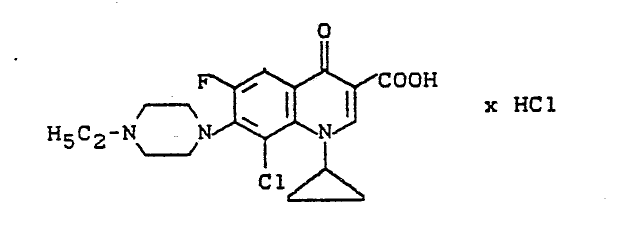

30 g (0,1 mmol) 8-Chlor-1-cyclopropyl-6,7-difluor-1,4-dihydro-4-oxo-3-chinolincarbonsäure werden in 250 ml Pyridin mit 35 g (0,3 mol) 1-Ethylpiperazin unter einer Stickstoffatmosphäre 1 Stunde unter Rückfluß erhitzt. Die Suspension wird abgekühlt, der Niederschlag abgesaugt mit Ethanol gewaschen und das Rohprodukt in 100 ml 6n-Salzsäure in der Hitze gelöst, filtriert und das Filtrat mit 500 ml Ethanol versetzt. Das beim Abkühlen ausfallende Kristallisat wird abgesaugt, mit Ethanol gewaschen und bei 80°C im Hochvakuum getrocknet. Man erhält 9,8 g (23 % Theorie) 8-Chlor-1-cyclopropyl-7-(4-ethyl-1-piperazinyl)-6-fluor-1,4-dihydro-4-oxo-3-chinolincarbonsäure Hydrochlorid vom Schmelzpunkt 245-248°C (unter Zersetzung).

### Beispiel 8

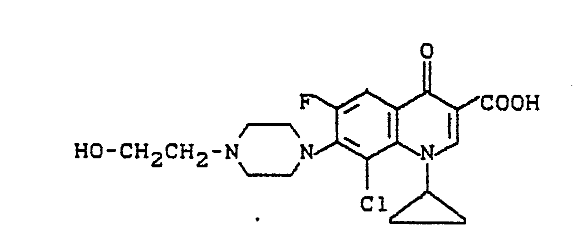

Eine Mischung aus 4,8 g (16 mmol) 8-Chlor-1-cyclopropyl-6,7-difluor-1,4-dihydro-4-oxo-3-chinolincarbonsäure und 7,8 g (60 mmol) N-(2-Hydroxyethyl)-piperazin in 40 ml Pyridin wird unter einer Stickstoffatmosphäre 2 Stunden unter Rückfluß erhitzt. Anschließend wird im Vakuum eingeengt, der Rückstand in Wasser aufgenommen und mit verdünnter Salzsäure auf pH 6 eingestellt. Der nach einigen Minuten ausgefallene Niederschlag wird abgesaugt, mit Wasser gewaschen und bei 70°C im Vakuum getrocknet. Man erhält 4,9 g (75 %) Rohprodukt vom Schmelzpunkt 218-220°C (unter Zersetzung); nach Umkristallisation aus Glykolmonomethylether isoliert man 2,8 g (43 %) 8-Chlor-1-cyclopropyl-6-fluor-1,4-dihydro-7-[4-(2-hydroxyethyl)-1-piperazinyl]-4-oxo-3-chinolincarbonsäure vom Schmelzpunkt 240-247°C (unter Zersetzung).

### Beispiel für eine erfindungsgemäße Tablette

Jede Tablette enthält:

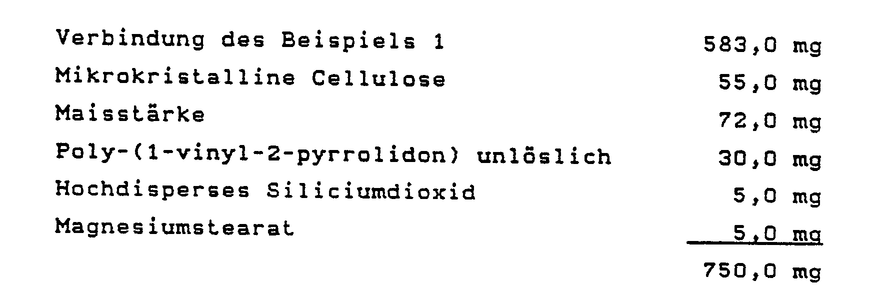

Die Lackhülle enthält:

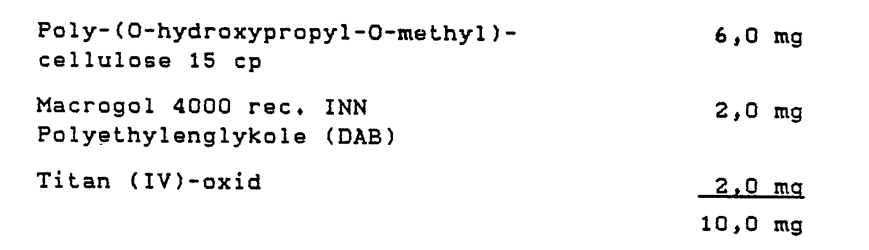

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen eine sehr breites Spektrum von Mikroorganismen wirksam. Mit ihrer Hilfe können gram-negative und gram-positive Bakterien und bakterienähnliche Mikroorganismen bekämpft sowie die durch dieser Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege); Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere); Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vor liegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutische wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Mengen Wirkstoffe auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

## Patentansprüche

1. 7-Amino-1-cyclopropyl-8-chlor-6-fluor-1,4-dihydro-4-oxo-3-chinolincarbonsäuren der Formel I 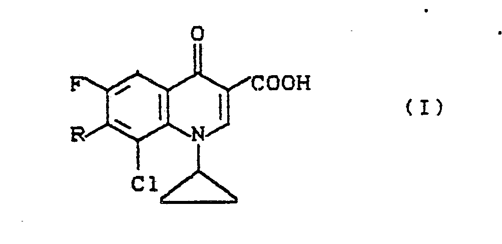 in welcher
R für 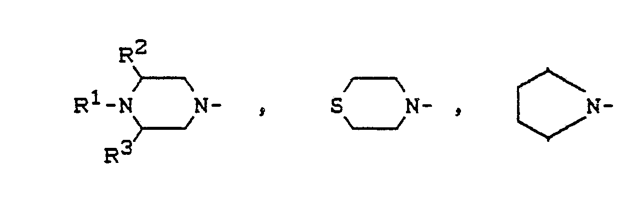 oder 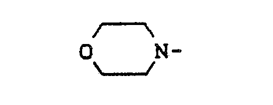
steht, wobei
R¹ H, CH₃, C₂H₅ oder HO-CH₂-CH₂- bedeutet und
R² für H, CH₃, C₂H₅, 2-Thienyl oder Phenyl und
R³ für H oder CH₃ stehen,
mit der Maßgabe, daß R nicht für 1-Piperazinyl, 4-Methyl-1-piperazinyl oder 3-Methyl-1-piperazinyl stehen kann sowie deren pharmazeutisch verwendbare Hydrate, Säureadditionssalze, Alkali-, Erdalkali- und Guanidiniumsalze.

2. 8-Chlor-cyclopropyl-6-fluor-1,4-dihydro-7-(cis-3,5-dimethyl-1-piperazinyl)-4-oxo-3-chinolincarbonsäure der Formel

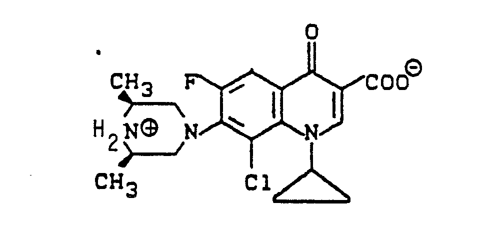

3. 8-Chlor-cyclopropyl-7-(4-ethyl-1-piperazinyl-6-fluor-1,4-dihydro-4-oxo-3-chinolincarbonsäure der Formel

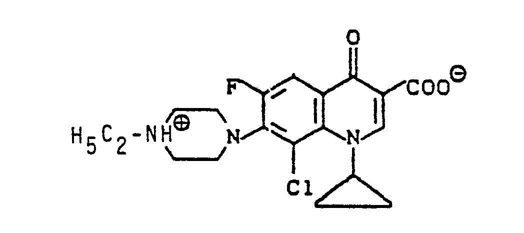

4. 8-Chlor-cyclopropyl-6-fluor-1,4-dihydro-7-[4-(2-hydroxyethyl)-1-piperazinyl]-4-oxo-3-chinolincarbonsäure der Formel

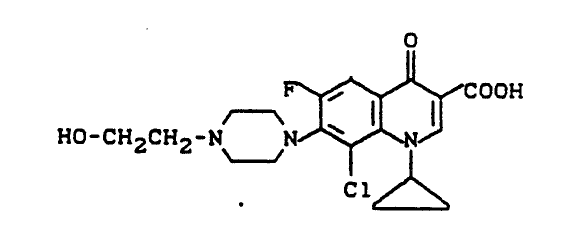

5. Verfahren zur Herstellung von 7-Amino-1-cyclopropyl-8-chlor-6-fluor-1,4-dihydro-4-oxo-3-chinolincarbonsäuren der Formel (I) 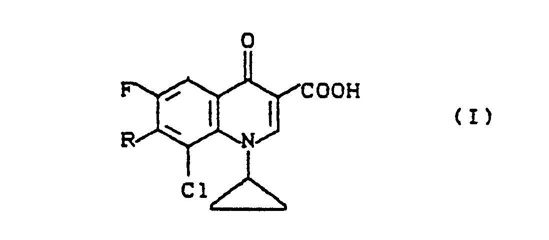 in welcher
R für 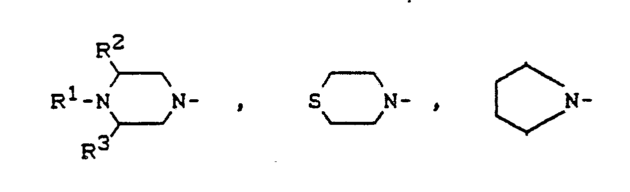 oder 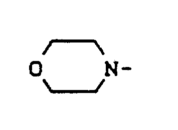
steht, wobei
R¹ H, CH₃, C₂H₅ oder HO-CH₂-CH₂- bedeutet und
R² für H, CH₃ C₂H₅, 2-Thienyl oder Phenyl und
R³ für H oder CH₃ stehen,
mit der Maßgabe, daß R nicht für 1-Piperazinyl-, 4-Methyl-1-piperazinyl- oder 3-Methyl-1-piperazinyl stehen kann sowie deren pharmazeutisch verwendbare Hydrate, Säureadditionssalze, Alkali-, Erdalkali- und Guanidiniumsalze, dadurch gekennzeichnet, daß man die 8-Chlor-1-cyclopropyl-6,7-difluor-1,4-dihydro-4-oxo- 3-chinolincarbonsäure der Formel (II), 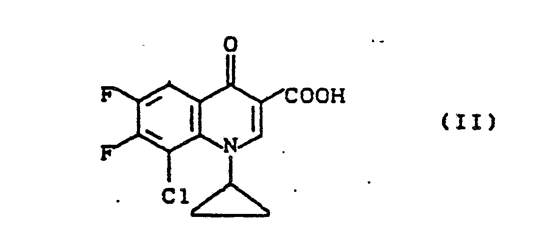 mit Aminen der Formel (III),
R - H (III)
in welcher
R die oben angegebene Bedeutung hat,
gegebenenfalls in Gegenwart von Säurebindern umsetzt.

6. Verfahren zur Herstellung von 7-Amino-1-cyclopropyl-8-chlor-6-fluor-1,4-dihydro-4-oxo-3-chinolincarbonsäuren der Formel (I) 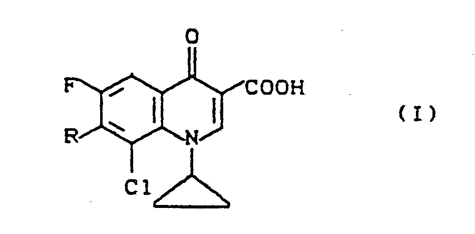 in welcher
R für 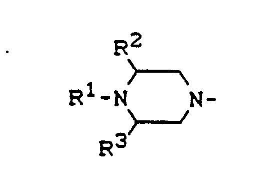 steht, wobei
R¹ H, CH₃, C₂H₅ oder HO-CH₂-CH₂- bedeutet und
R² für H, CH₃, C₂H₅, 2-Thienyl oder Phenyl und
R³ für H oder CH₃ stehen,
mit der Maßgabe, daß R nicht für 1-Piperazinyl-, 4-Methyl-1-piperazinyl- oder 3-Methyl-1-piperazinyl stehen kann sowie deren pharmazeutisch verwendbare Hydrate, Säureadditionssalze, Alkali-, Erdalkali- und Guanidiniumsalze, dadurch gekennzeichnet, daß man eine 7-(1-Piperazinyl)-3-chinoloncarbonsäure der Formel (IV) 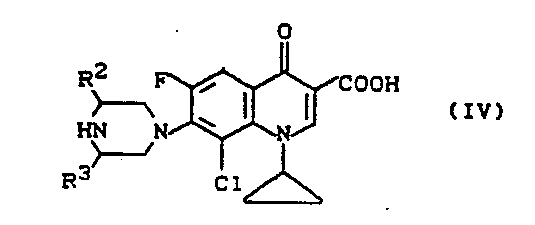 in welcher
R² und R³ die oben angegebene Bedeutung haben,
mit Verbindungen der Formel (V)
R¹X (V)
in welcher
R¹ die oben angegebene Bedeutung hat, jedoch nicht Wasserstoff sein kann, und
X Fluor, Chlor, Brom, Iod bedeutet,
gegebenenfalls in Gegenwart von Säurebindern umsezt.
